# EUROPEAN PATENT APPLICATION

(11) **EP 4 813 215 A1**
(43) Date of publication of application: **30.09.2026**
(21) Application number: 23959542.4
(22) Date of filing: 23.11.2023
(51) Int. Cl.: A23L 7/104, C12N 1/14, A23L 7/17, A23L 7/191, A23L 7/10, A23L 33/105, C12R 1/07

(54) **PUFFED GRAIN-FERMENTED ENZYME HAVING SUPEROXIDE DISMUTASE ACTIVITY AND CATALASE ACTIVITY, PRODUCTION METHOD THEREFOR, AND FOOD COMPOSITION COMPRISING SAME**

(30) Priority: 21.11.2023 KR 20230162534
(71) Applicant: NPK INC., Jeollanam-do 57309 (KR)
(72) Inventor: SHON, Sung Oh, Gwangju 61092 (KR); JO, Woon Hee, Jeollanam-do 57231 (KR); JEON, So Heon, Gwangju 62279 (KR)
(74) Representative: Perreaud, Jérémie
(86) International application number: PCT/KR2023/018975
(87) International publication number: WO 2025/110285

(57) **Abstract**

The present invention relates to a puffed grain-fermented enzyme having excellent superoxide dismutase activity and catalase activity, formed by fermenting puffed grain with a *Bacillus amyloliquefaciens* strain, a method for producing the same, and a food composition including the same. According to the present invention, since no food additive enzyme or coating agent is added, the puffed grain-fermented enzyme provides excellent taste and economic efficiency while exhibiting superior superoxide dismutase and catalase activities.

## Description

### [Technical Field]

The present invention relates to a puffed grain-fermented enzyme having superoxide dismutase (SOD) activity and catalase activity, a method for producing the same, and a food composition including the same. More specifically, the present invention relates to a puffed grain-fermented enzyme having excellent superoxide dismutase activity and catalase activity, obtained by fermenting puffed grain with a *Bacillus amyloliquefaciens* strain, a method for producing the same, and a food composition including the same.

### [Background Art]

One of the most important elements of a human body is an enzyme, which is necessary for the body to properly perform its functions. If enzymes are absent in the body, many physiological functions such as breathing, digestion, and nutrient synthesis may be impaired.

When the body is young, it can sufficiently synthesize and supply enzymes. However, modern unhealthy eating habits such as smoking, drinking, high-fat diets, and excessive sugar intake stress cells, leading to cell death and a decrease in enzyme synthesis. As a result, the remaining cells must produce more digestive enzymes, causing a vicious cycle that ultimately reduces the body's overall endurance.

Therefore, taking enzyme supplements to improve digestion may help decompose ingested food, reduce enzyme synthesis in the body, and contribute not only to maintaining stomach health but also to improving biological rhythm.

The term "enzyme food" refers to a product made by culturing edible microorganisms on plant-based raw materials so that contains a large amount of enzymes, extracting the enzyme-containing portion from food, or processing a product using the extracted portion as a main ingredient.

Such enzyme foods are known to contain various trace elements and physiologically active substances that aid digestion and absorption, through the generation of various bioactive compounds and nutrients and the proliferation of beneficial microorganisms during the fermentation and aging processes involving the food's own enzymes and microorganisms.

A grain-fermented enzyme is a general term for enzyme foods produced by culturing edible microorganisms on grain raw materials to contain a large amount of enzymes. Prior art discloses technologies using one grain among brown rice, soybeans, barley, and mixed grains (wheat, corn, and adlay) as the grain raw material (Korean Patent Publication No. 10-1855125), or using rice and other rice grains; cereals such as barley, wheat, rye, and oats; or miscellaneous grains such as millet, corn, foxtail millet, buckwheat, and adlay (Korean Patent Publication No. 10-2088758).

However, no prior art has disclosed a puffed grain-fermented enzyme having excellent superoxide dismutase activity and catalase activity, formed by fermenting puffed grains with a *Bacillus amyloliquefaciens* strain, as in the present invention.

### [Prior Art Document]

### [Patent Document]

(Patent Document 001) Korean Patent Publication No. 10-1855125
(Patent Document 002) Korean Patent Publication No. 10-2088758

### [Summary of Invention]

### [Problems to be Solved by Invention]

An object of the present invention is to provide a puffed grain-fermented enzyme having superoxide dismutase activity and catalase activity, formed by fermenting puffed grain with a *Bacillus amyloliquefaciens* strain.

Another object of the present invention is to provide a method for producing a puffed grain-fermented enzyme having superoxide dismutase activity and catalase activity by fermenting puffed grain with a *Bacillus amyloliquefaciens* strain.

A further object of the present invention is to provide a food composition including a puffed grain-fermented enzyme having superoxide dismutase activity and catalase activity, formed by fermenting puffed grain with a *Bacillus amyloliquefaciens* strain.

### [Means for Solving Problems]

To achieve the above-described objects, the present invention provides a puffed grain-fermented enzyme formed by fermenting puffed grain with a *Bacillus amyloliquefaciens* strain and having superoxide dismutase activity and catalase activity.

As one embodiment of the present invention, the grain may be at least one selected from the group consisting of defatted soybeans, oats, brown rice, white soybeans, barley, sorghum, linseed, rice germ, sword beans, sword bean hulls, wheat, rye, and millet.

As one embodiment of the present invention, the strain may be *Bacillus amyloliquefaciens* NPKE6 deposited under the accession number KCCM13257P.

As one embodiment of the present invention, the enzyme has a superoxide dismutase activity of 37 to 88% at a concentration of 10 to 100 mg/mL of the puffed grain-fermented enzyme, and a catalase activity of 1439 to 3276 mU/mL at a concentration of 5 to 100 mg/mL of the puffed grain-fermented enzyme, wherein 1 Unit of the catalase activity is an amount that decomposes 1 µmole of H₂O₂ at pH 7.0 and 25 °C for 1 minute.

As one embodiment of the present invention, the grain may be at least one selected from the group consisting of defatted soybeans, oats, brown rice, white soybeans, barley, sorghum, linseed, rice germ, sword beans, sword bean hulls, wheat, rye, and millet.

In order to achieve the above objects, the present invention provides a method for producing a puffed grain-fermented enzyme, including: (a) grinding puffed grain; (b) immersing the puffed grain in water; (c) inoculating the grain with *Bacillus amyloliquefaciens* strain and then mixing; and (d) fermenting the grain inoculated with the strain.

As one embodiment of the present invention, the production method may further include, before step (a), putting grains into an extrusion puffer and puffing them at a temperature of 140 to 160 °C and a pressure of 80 to 120 bar to obtain puffed grains, and may further include, after step (d), hot-air drying and powdering the grains.

As one embodiment of the present invention, the grain may be at least one selected from the group consisting of defatted soybeans, oats, brown rice, white soybeans, barley, sorghum, linseed, rice germ, sword beans, sword bean hulls, wheat, rye, and millet.

As one embodiment of the present invention, the strain may be *Bacillus amyloliquefaciens* NPKE6 deposited under the accession number KCCM13257P.

To achieve the above objects, the present invention provides a food composition including the puffed grain-fermented enzyme.

As one embodiment of the present invention, the food composition may be an enzyme food.

### [Advantageous Effects]

According to the present invention, since no food additive enzyme or coating agent is added, there is an advantage in that a puffed grain-fermented enzyme having excellent taste and economic efficiency and superoxide dismutase activity and catalase activity can be provided.

Further, according to the present invention, there is an advantage in that it is possible to provide a health-oriented superfood by adding only the puffed grain-fermented enzyme and plant-based raw materials, thereby complying with the recommendations of the Ministry of Food and Drug Safety and utilizing grain-based ingredients.

### [Brief Description of Drawings]

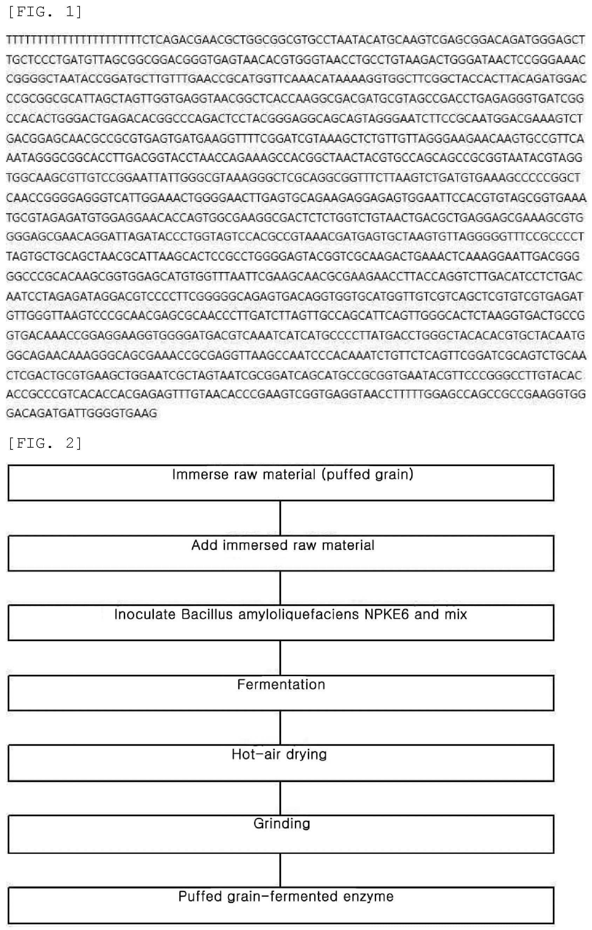

FIG. 1 shows the 16S rRNA base sequence of *Bacillus amyloliquefaciens* NPKE6 strain (KCCM13257P) of the present invention.
FIG. 2 is a manufacturing process diagram of a puffed grain-fermented enzyme according to one embodiment of the present invention.
FIG. 3 shows the DPPH radical scavenging ability of a puffed grain-fermented enzyme according to one embodiment of the present invention.
FIG. 4 shows the superoxide dismutase (SOD) activity of the puffed grain-fermented enzyme according to one embodiment of the present invention.
FIG. 5 shows the catalase activity of the puffed grain-fermented enzyme according to one embodiment of the present invention.

### [Mode for Carrying out Invention]

A first embodiment of the present invention relates to a puffed grain-fermented enzyme formed by fermenting puffed grain with a *Bacillus amyloliquefaciens* strain and having superoxide dismutase activity and catalase activity.

The strain may be *Bacillus amyloliquefaciens* NPKE6 deposited under the accession number KCCM13257P, and the strain may have superoxide dismutase (SOD) activity and catalase activity.

Th*e Bacillus amyloliquefaciens* NPKE6 strain may additionally have DPPH radical scavenging activity, but is not limited thereto.

Further, the puffed grain-fermented enzyme of the present invention has a superoxide dismutase activity of 37 to 88% at a concentration of 10 to 100 mg/mL of the puffed grain-fermented enzyme, and a catalase activity of 1439 to 3276 mU/mL at a concentration of 5 to 100 mg/mL of the puffed grain-fermented enzyme, wherein 1 Unit of the catalase activity is an amount that decomposes 1 µmole of H₂O₂ at pH 7.0 and 25 °C for 1 minute.

In one embodiment of the present invention, the grain may be at least one selected from the group consisting of defatted soybeans, oats, brown rice, white soybeans, barley, sorghum, linseed, rice germ, sword beans, sword bean hulls, wheat, rye, and millet, but is not limited thereto.

A second embodiment of the present invention relates to a method for producing a puffed grain-fermented enzyme, which includes: the steps of: (a) grinding puffed grain; (b) immersing the puffed grain in water; (c) inoculating the grain with *Bacillus amyloliquefaciens* strain and then mixing; and (d) fermenting the grain inoculated with the strain.

The method for producing the puffed grain-fermented enzyme of the present invention may further include, before step (a), putting grain into an extrusion puffer and puffing them at a temperature of 140 to 160 °C, preferably 145 to 155 °C, and more preferably 150 °C, and a pressure of 80 to 120 bar, preferably 90 to 110 bar, and more preferably 100 bar, to obtain puffed grain.

Further, the method may further include, after step (d), hot-air drying and powdering the grains, however, the powdered product may be formulated into another dosage form as needed.

In the method for producing the puffed grain-fermented enzyme of the present invention, the grain may be at least one selected from the group consisting of defatted soybeans, oats, brown rice, white soybeans, barley, sorghum, linseed, rice germ, sword beans, sword bean hulls, wheat, rye, and millet, but is not limited thereto.

In the method for producing the puffed grain-fermented enzyme of the present invention, the strain may be *Bacillus amyloliquefaciens* NPKE6 deposited under the accession number KCCM13257P, but is not limited thereto.

A third embodiment of the present invention relates to a food composition including the puffed grain-fermented enzyme.

The food composition may preferably be an enzyme food, but is not limited thereto. In addition to the puffed grain-fermented enzyme, the food composition may further include at least two kinds of fermented mixed grains selected from the group consisting of brown rice, white soybeans, sorghum, and barley; at least two kinds of grain mixed powders selected from the group consisting of roasted brown rice, roasted soybeans, and papaya extract; and potato extract powder, but is not limited thereto.

The food composition may include 20 to 40% by weight ("wt%") of the fermented grain, 50 to 70 wt% of the fermented mixed grain, 1 to 5 wt% of the grain mixed powder, and 1 to 5 wt% of the potato extract powder based on the total weight thereof, but is not limited thereto.

The food composition may preferably include 30 to 35 wt% of the puffed grain-fermented enzyme, 55 to 65 wt% of the fermented mixed grain, 2 to 4 wt% of the grain mixed powder, and 2 to 4 wt% of the potato extract powder based on the total weight thereof, but is not limited thereto.

The food composition may more preferably include 33 to 34 wt% of the puffed grain-fermented enzyme, 60 to 62 wt% of the fermented mixed grain, 2.5 to 3.5 wt% of the grain mixed powder, and 2.5 to 3.5 wt% of the potato extract powder based on the total weight thereof, but is not limited thereto.

Brown rice, which may be included in the fermented mixed grain, is rice that has been dried and threshed after harvesting, with the husk removed by a machine equipped with rubber rollers, and is known to be effective in preventing adult diseases.

The white soybeans, which may be included in the fermented mixed grains, are also called meju beans. In terms of nutrition, they contain particularly high amounts of components such as lecithin, saponin, isoflavone, and trypsin inhibitor. These components have anticancer effects, reduce blood cholesterol, prevent obesity by inhibiting fat synthesis, activate intestinal movement through intestinal regulation, and prevent constipation by facilitating bowel movements.

Sorghum, which may be included in the fermented mixed grains, is low in fat and calories, making it suitable as a diet food. It has the effect of improving cholesterol levels and decomposing waste in the intestines. Proanthocyanidin contained in sorghum strengthens the immune function of the bladder and helps alleviate inflammation by reducing oxidative stress in cells.

Barley, which may be included in the fermented mixed grains, is rich in dietary fiber and helps relieve constipation. It also contains abundant polyphenol compounds that contribute to antioxidant effects and immunity improvement, and tocotrienol, which lowers cholesterol levels and helps prevent vascular diseases.

The papaya extract, which may be included in the grain mixed powder, contains papain, a type of protein-decomposing enzyme extracted from papaya fruit. Papain is one of the powerful digestive enzymes found in nature and is known to have various effects, such as helping activate digestive functions, suppressing stomach bloating, promoting diuresis, and exhibiting anti-inflammatory effects.

In the potato extract powder included in the food composition, the potato is an alkaline food that protects the stomach by protecting and improving mucous membranes as well as ulcers caused by excessive gastric acid. It contains a large amount of vitamin C (similar to that of spinach or tangerines). The vitamin C in potatoes is trapped in starch, making it relatively resistant to heat. Potatoes also contain a high potassium content of 410 to 485 mg per 100 g compared to other vegetables or foods. In addition, the starch in potatoes contains a large amount of pectin, a type of dietary fiber, which helps improve intestinal function and promotes smooth bowel movements.

Hereinafter, in order to facilitate understanding of the present invention, examples and the like will be described in detail. However, the examples according to the present invention may be modified in various forms, and the scope of the present invention should not be construed as being limited to the following examples. The examples of the present invention are provided to more fully describe the present invention to those of ordinary skill in the art.

### <Example 1> Identification of Bacillus amyloliquefaciens NPKE6 strain

### 1. Base sequence analysis of Bacillus amyloliquefaciens NPKE6 strain

DNA extraction and 16S rRNA sequence analysis were entrusted to Macrogen. Bacterial identification was performed using the 16S rRNA sequence through the Basic Local Alignment Search Tool (BLAST) search engine of the National Center for Biotechnology Information (NCBI, www.ncbi.nlm.nih.gov).

As a result of analyzing the 16S rRNA base sequence for identification and classification of the isolated strain, the base sequence of Sequence No. 1 was obtained.

### 2. Identification of biochemical characteristics of Bacillus amyloliquefaciens NPKE6 strain

Biochemical characteristics of *Bacillus amyloliquefaciens* NPKE6 strain were investigated using API 50CH (bioMérieux Co., France), which is used for Bacillus identification. The API kit was used according to the manufacturer's instructions, and the results are shown in Table 1 below.

The *Bacillus amyloliquefaciens* NPKE6 strain was inoculated into TSB (casein 17.0 g, soybean meal 3.0 g, NaCl 5.0 g, dipotassium phosphate 2.5 g, dextrose 2.5 g, final pH 7.3 ± 0.2 at 25 °C) liquid medium and cultured at 37 °C. The culture was dispensed into Eppendorf tubes, centrifuged at 10,000 rpm for 5 minutes, and the cells were harvested and washed once with sterile saline (0.85%). The cells were then suspended in sterile saline and inoculated into ampoules of aseptically opened API 50CH medium. After pipetting to mix evenly, 150 µL of the mixture was dispensed into the microtubes of each test strip.

After dispensing, the API kit was incubated at 37 °C for 24 hours, and color changes were observed. In Table 1 below, "+" indicates utilization of the substrate, and "-" indicates no utilization.

**[TABLE 1]**

| Characteristics | Result | Characteristics | Result |
|---|---|---|---|
| Glycerol | - | Salicin | - |
| Erythritol | - | Cellobiose | - |
| D-arabinose | - | Maltose | - |
| L-arabinose | - | Lactose | - |
| Ribose | - | Melibiose | - |
| D-xylose | - | Sucrose | + |
| L-xylose | - | Trehalose | - |
| Adonitol | - | Inulin | - |
| Methyl-β-D-xylopyranoside | - | Melezitose | - |
| Galactose | - | Raffinose | - |
| Glucose | | Starch | |
| Fructose | - | Glycogen | - |
| Mannose | - | Xylitol | - |
| Sorbose | - | Gentiobiose | - |
| Rhamnose | - | D-furanose | - |
| Dulsitol | - | D-lyxose | - |
| Inositol | - | D-tagatose | - |
| Mannitol | - | D-fucose | - |
| Sorbitol | - | L-fucose | - |
| Methyl-α-D-mannopyranoside | - | D-arabitol | - |
| Methyl-α-D-glucoside | - | L-arabitol | - |
| N-acetyl-glucosamine | - | Gluconate | - |
| Amygdalin | - | 2-keto-gluconate | - |
| Arbutin | - | 5-keto-gluconate | - |
| Esculin | + | | |

As a result of identification, based on the comprehensive biochemical characteristics described above, the selected NPKE6 strain was identified as *Bacillus amyloliquefaciens* and designated as *Bacillus amyloliquefaciens* NPKE6. The strain was deposited with the Korean Culture Center of Microorganisms (KCCM) on October 31, 2022, and was assigned the accession number KCCM13257P.

### <Example 2> Preparation of grain-fermented enzyme using Bacillus amyloliquefaciens NPKE6 strain

### 1. Culture of strains

*Bacillus amyloliquefaciens* NPKE6 was inoculated (1.0% v/v) into BM medium containing D-glucose monohydrate (13.5 g/L), hydrolysed soy protein (3 g/L), yeast extract (3 g/L), sodium carbonate (0.3 g/L), and magnesium sulfate heptahydrate (0.2 g/L), and cultured at 30 °C for 24 hours.

### 2. Preparation of puffed grains

After weighing the grains, 18% of water was added and mixed. The grains were puffed into 2.5 cm × 2.5 cm particles under conditions of 150 °C and 100 bar, using cooling water at 30 °C.

### 3. Preparation of puffed grain-fermented enzyme

After weighing the puffed grains, nine times the amount of sterile water at 70 °C was added. After immersing for 20 minutes, the unabsorbed sterile water was removed. 2.5 kg of the immersed material was placed per tray, followed by inoculating the *Bacillus amyloliquefaciens* NPKE6 inoculum, which had been pre-cultured in BM medium at 30 °C and 150 rpm for 24 hours, at an inoculation rate of 2% (v/w) based on the immersed material.

Fermentation was carried out at 30 °C and a relative humidity of 80% or higher for 24 hours. Thereafter, the product was dried with hot air at 70 °C for 24 hours and ground to a particle size of 60 mesh using a multipurpose grinder to obtain the grain-fermented enzyme.

### <Example 3> Analysis of enzyme activity of puffed grain-fermented enzyme

### 1. Confirmation of DPPH (2,2-diphenyl-1-picrylhydrazyl) radical scavenging ability

The puffed grain-fermented enzyme produced in Example 2 was diluted with sterile water according to concentration, centrifuged at 4,000 rpm for 20 minutes, and filtered through a 0.2 µm filter to obtain a puffed grain-fermented enzyme filtrate.

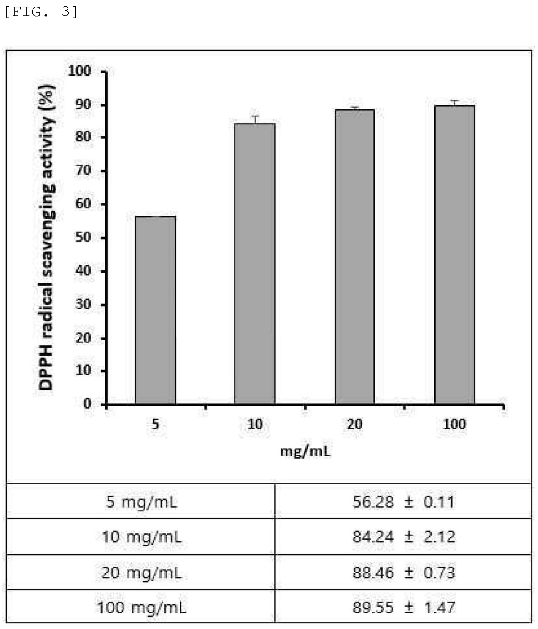

Eighty microliters (80 µL) of each diluted sample was mixed with 500 µL of 0.2 mM DPPH (2,2-diphenyl-1-picrylhydrazyl) solution and 420 µL of ethanol. The absorbance was then measured at 517 nm, and the DPPH radical scavenging activity was calculated using the following formula. The results are shown in FIG. 3. 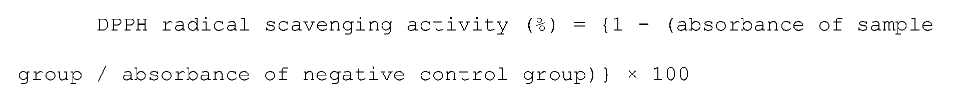 DPPH radical scavenging activity (%) = {1 - (absorbance of sample group / absorbance of negative control group)} × 100

As shown in FIG. 3, the DPPH radical scavenging activity of the puffed grain-fermented enzyme increased in a concentration-dependent manner.

### 2. SOD activity

The SOD activity of the puffed grain-fermented enzyme prepared in Example 2 was measured using the EZ-SOD Assay Kit (DoGen, Korea) according to the manufacturer's instructions.

Specifically, the puffed grain-fermented enzyme was diluted with sterile water according to concentration, centrifuged at 4,000 rpm for 20 minutes, and filtered through a 0.2 µm filter to obtain a puffed grain-fermented enzyme filtrate. As shown in Table 2 below, 20 µL of each concentration-diluted sample was added to each sample well and the well of Blank 2. At this time, 20 µL of ddH₂O was added to each of the Blank 1 and Blank 3 wells.

Two hundred microliters (200 µL) of WST working solution was added to each well. Twenty microliters (20 µL) of dilution buffer was added to each of the Blank 2 and Blank 3 wells. Using a multi-channel pipette, 20 µL of enzyme working solution was added to each of the Blank 1 and sample wells, followed by gentle mixing. A 96-well plate was then incubated at 37 °C for 20 minutes.

**[TABLE 2]**

| | Blank 1 | Blank 2 | Blank 3 | Test sample |
|---|---|---|---|---|
| Sample | - | 20 Ml | - | 20 µL |
| ddH₂O | 20 µL | - | 20 µL | - |
| WST working solution | 200 µL | 200 µL | 200 µL | 200 µL |
| Diluted buffer | - | 20 µL | 20 µL | - |
| Enzyme working solution | 20 µL | - | - | 20 µL |
| Total volume | 240 µL | 240 µL | 240 µL | 240 µL |

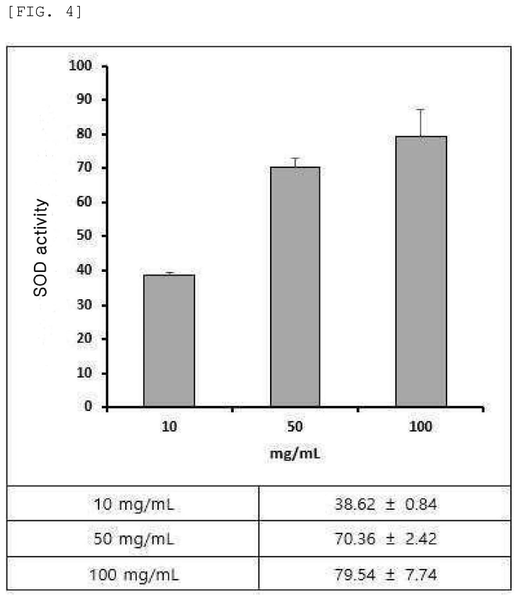

After measuring the absorbance at 450 nm, the SOD activity of the puffed grain-fermented enzyme was calculated using the following formula, and the results are shown in FIG. 4. 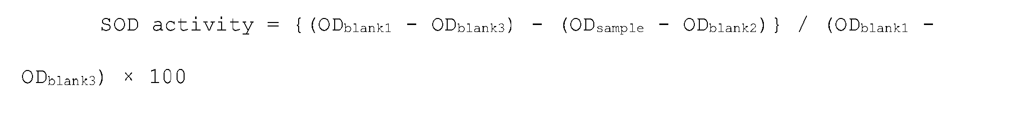 SOD activity = {(ODblank1 - ODblank3) - (ODsample - ODblank2)} / (ODblank1 - ODblank3) × 100

As shown in FIG. 4, the SOD activity of the puffed grain-fermented enzyme increased in a concentration-dependent manner.

### 3. Catalase activity

The catalase activity of the puffed grain-fermented enzyme prepared in Example 2 was measured using the EZ-Catalase Assay Kit (DoGen, Korea) according to the manufacturer's instructions.

Specifically, the puffed grain-fermented enzyme was diluted with sterile water according to concentration, centrifuged at 4,000 rpm for 20 minutes, and filtered through a 0.2 µm filter to obtain a puffed grain-fermented enzyme filtrate. Then, 25 µL of each concentration-diluted sample and 25 µL of 40 µM H₂O₂ solution were added to the 96-well plate.

After incubating the plate for 30 minutes at room temperature under light-shielded conditions, 50 µL of Oxi-Probe/HRP working solution, prepared by mixing 30 µL of 10 mM Oxi-Probe, 12 µL of 100 U/mL horseradish peroxidase (HRP) and 3 mL of 1X reaction buffer, was added to each well of the plate where the reaction had been completed.

The plate was then incubated for 30 minutes at 37 °C under light-shielded conditions, and the absorbance was measured at 560 nm. The catalase activity at each concentration was calculated by substituting the measured value into the standard calibration curve (y = 23673x - 224.98, R² = 0.999).

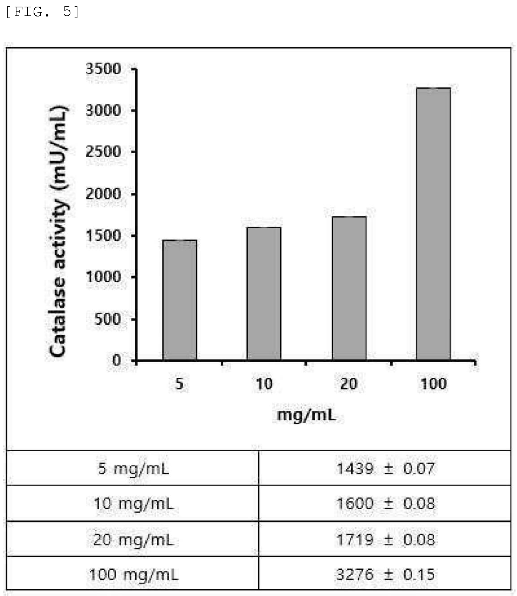

The catalase activity (unit: mU/mL) of the puffed grain-fermented enzyme is shown in FIG. 5. As shown in FIG. 5, the catalase activity of the puffed grain-fermented enzyme increased in a concentration-dependent manner.

### <Example 4> Measurement of the number of Bacillus amyloliquefaciens viable cells in puffed grain-fermented enzyme

The sample was analyzed using a dilution solution prepared by serially diluting the sample 10-fold with physiological saline. The number of viable cells was measured by spreading 100 µL of the prepared dilution solution onto a sterilized and dried PCA solid medium, followed by incubation at 37 °C for 16 hours, after which the colony-forming units were counted.

The measured viable cell counts of **Bacillus amyloliquefaciens** are shown in Table 3 below.

**[TABLE 3]**

| Division | Viable cell number (CFU/g) |
|---|---|
| Defatted soybean hull | 3.0 × 10⁸ |
| Puffed defatted soybean hull | 3.7 × 10⁸ |

While specific aspects of the present invention have been described in detail above, it will be apparent to those skilled in the art that such detailed descriptions are merely preferred embodiments, and that the scope of the present invention is not limited thereby.

Accordingly, the substantial scope of the present invention shall be defined by the appended claims and their equivalents. Simple modifications or variations of the present invention can be readily made by those skilled in the art, and all such modifications or variations shall be considered to fall within the scope of the present invention.

### [Accession Number]

Name of Depositor: Korea Culture Collection of Microorganisms (KCCM)
Address of Depository: Yurim Building, 45 Hongjenae-2ga gil, Seodaemun-gu, Seoul
Deposit Date: 2022-10-31
Deposit Number: KCCM 13257P

BUDAPEST TREATY ON THE INTERNATIONAL RECOGNITION OF THE DEPOSIT OF
MICROORGANISMS FOR THE PURPOSES OF PATENT PROCEDURE
INTERNATIONAL FORM

Depositor: Naturepure Korea | Receipt issued pursuant to Rule
61 Eco-gil, Damyang-eup, Damyang-gun | 7.1 by the International Deposi-
Jeollanam-do, Korea | tary Authority

I. IDENTIFICATION OF THE MICROORGANISM
Identification reference given by the Depositor:
Bacillus amyloliquefaciens NPKE6 | Accession number given by the International
Depositary Authority:
KCCM13257P
II. SCIENTIFIC DESCRIPTION AND/OR PROPOSED TAXONOMIC DESIGNATION
The microorganism identified under I above was accompanied by:
() scientific description
() proposed taxonomic designation
(Mark with a cross where applicable)
III. RECEIPT AND ACCEPTANCE
This International Depositary Authority accepts the microorganism identified under I above, which
was received by it on October 31, 2022
IV. RECEIPT OF REQUEST FOR CONVERSION
The microorganism identified under I above was received by this International Depositary Authority,
and a request to convert the original deposit to a deposit under the Budapest Treaty was received by
it.
V. INTERNATIONAL DEPOSITARY AUTHORITY
Name: Korea Culture Collection of
Microorganisms (KCCM)
Address: Yurim Building, 45 Hongjenae-2ga gil,
Seodaemun-gu, Seoul | Signature(s) of person(s) having the power to
represent the International Depositary Authority
or of authorized official(s):
Date: October 11, 2023

* It is to certify that the above translation is identical to the contents of the original copy

November 21, 2023
Attorney HWANG, E-Nam

## Claims

1. A puffed grain-fermented enzyme formed by fermenting puffed grain with a *Bacillus amyloliquefaciens* strain and having superoxide dismutase activity and catalase activity.

2. The enzyme according to claim 1, wherein the strain is *Bacillus amyloliquefaciens* NPKE6 deposited under the accession number of KCCM13257P.

3. The enzyme according to claim 1, wherein the enzyme has a superoxide dismutase activity of 37 to 88% at a concentration of 10 to 100 mg/mL of the puffed grain-fermented enzyme, and
a catalase activity of 1439 to 3276 mU/mL at a concentration of 5 to 100 mg/mL of the puffed grain-fermented enzyme,
wherein 1 Unit of the catalase activity is an amount that decomposes 1 µmole of H₂O₂ at pH 7.0 and 25 °C for 1 minute.

4. The enzyme according to claim 1, wherein the grain is at least one selected from the group consisting of defatted soybeans, oats, brown rice, white soybeans, barley, sorghum, linseed, rice germ, sword beans, sword bean hulls, wheat, rye, and millet.

5. A method for producing puffed grain-fermented enzyme, comprising:
(a) grinding puffed grain;
(b) immersing the puffed grain in water;
(c) inoculating the grain with *Bacillus amyloliquefaciens* strain and then mixing; and
(d) fermenting the grain inoculated with the strain.

6. The method according to claim 5, further comprising: before step (a), putting grains into an extrusion puffer and puffing them at a temperature of 140 to 160 °C and a pressure of 80 to 120 bar to obtain puffed grains, and
further comprising, after step (d), hot-air drying and powdering the grains.

7. The method according to claim 5, wherein the grain is at least one selected from the group consisting of defatted soybeans, oats, brown rice, white soybeans, barley, sorghum, linseed, rice germ, sword beans, sword bean hulls, wheat, rye, and millet.

8. The method according to claim 5, wherein the strain is *Bacillus amyloliquefaciens* NPKE6 deposited under the accession number of KCCM13257P.

9. A food composition comprising the puffed grain-fermented enzyme according to any one of claims 1 to 4.

10. The food composition according to claim 9, wherein the food composition is an enzyme food.
